# EUROPEAN PATENT APPLICATION

(11) **EP 4 424 641 A1**
(43) Date of publication of application: **04.09.2024**
(21) Application number: 22904126.4
(22) Date of filing: 01.12.2022
(51) Int. Cl.: C01G 9/02, A61Q 17/04, A61K 8/27, C09C 3/12

(54) **ZINC OXIDE PARTICLES EACH HAVING SURFACE COATED WITH ORGANOSILICON, METHOD FOR PRODUCING SAME, COSMETIC PREPARATION, DISPERSION, HEAT DISSIPATING FILLER AND RESIN COMPOSITION**

(30) Priority: 06.12.2021 JP 2021198077
(71) Applicant: Sakai Chemical Industry Co., Ltd., Sakai-shi, Osaka 590-8502 (JP)
(72) Inventor: YOSHIDA, Ryohei, Iwaki-shi, Fukushima 971-8183 (JP); KOBAYASHI, Keita, Sakai-shi, Osaka 590-0985 (JP); IWASAKI, Ryo, Iwaki-shi, Fukushima 971-8183 (JP)
(74) Representative: Isarpatent
(86) International application number: PCT/JP2022/044351
(87) International publication number: WO 2023/106195

(57) **Abstract**

To provide zinc oxide particles having a surface coated with organosilicon, the particles exhibiting a high dispersibility and excellent water repellency. Zinc oxide particles having a surface coated with organosilicon, the zinc oxide particles having a primary particle size of 0.1 µm or less as measured based on a transmission electron micrograph, and a median size based on a volume of 0.15 µm or less, where the median size is determined by charging the powder in isopropyl alcohol, dispersing the powder by application of ultrasonic wave with a rated output of 600 W and an oscillation amplitude of 100% generated by an ultrasonic homogenizer at a normal temperature for 30 seconds, and measuring the dispersion by a laser diffraction scattering method.

## Description

### Technical Field

The present invention relates to zinc oxide particles having a surface coated with organosilicon, a method for producing the same, a cosmetic preparation, a dispersion, a heat dissipating filler, and a resin composition.

### Background Art

Inorganic powders such as zinc oxide and titanium oxide microparticulates having a particle size of a few tens to 100 nm have been widely used as an additive for a sunscreen cosmetic preparation, ink for outdoor use, a food packaging material, and the like because of its characteristics. When such a powder is untreated, because the powder has a hydrophilic surface, the powder may run away due to sweat or rain. Thus, especially for use in a cosmetic preparation or the like, such a powder is often used after subjecting a particle surface to a water-repellent treatment with silicone, a metallic soap, or the like. Furthermore, since such a powder is often blended with an oily component in a cosmetic preparation, it is important to perform a water-repellent treatment uniformly for each particle to enhance dispersibility in the oily component.

However, known zinc oxide microparticulates having a particle size of 100 nm or less have high aggregability and almost no such zinc oxide microparticulates exist in a form of a primary particle. This results in subjecting aggregated zinc oxide particles to a water-repellent treatment, and thus it is difficult to form a uniform coating layer on each particle. Furthermore, in a case where such water-repellent treated zinc oxide particles are blended in an oily agent, a high dispersing force or a dispersing agent in a large amount was required to disperse the particles to a degree that is close to a primary particle state. Furthermore, when the aggregated particles are loosened due to the dispersing, an untreated surface is exposed, and thus problems such as thickening or precipitation of the particles or breaking of an emulsified system occur.

Meanwhile, in a powder in which the particles exist in a form of primary particles, the powder readily passes through a filter cloth or the like having air-permeability, and thus an amount of powder leakage tends to be greater. In addition, the bulk specific gravity also increases, thus the powder becomes soft, and the handleability becomes poor.

From the above, it is considered that water-repellent treated zinc oxide particles having a uniform coating layer, typically in a weakly aggregated state, and being readily dispersible by a weak impact are the most desirable.

Patent Document 1 describes zinc oxide having excellent dispersibility. Such zinc oxide has a low aggregability and achieves high independency and dispersibility of particles. However, the zinc oxide particles described in the document are not subjected to a surface treatment.

### Citation List

### Patent Documents

Patent Document 1: WO 2012/147888

### Summary of Invention

### Technical Problem

An object of the present invention is to provide zinc oxide particles having a surface coated with organosilicon, the particles exhibiting a high dispersibility and excellent water repellency.

### Solution to Problem

An embodiment of the present invention relates to zinc oxide particles having a surface coated with organosilicon, the zinc oxide particles having a primary particle size of 0.1 µm or less as measured based on a transmission electron micrograph, and a median size based on a volume of 0.15 µm or less, where the median size is determined by charging the powder in isopropyl alcohol, dispersing the powder by application of ultrasonic wave with a rated output of 600 W and an oscillation amplitude of 100% generated by an ultrasonic homogenizer at a normal temperature for 30 seconds to obtain a dispersion, and measuring the dispersion by a laser diffraction scattering method.

A median size, based on a volume, determined by charging the powder in isopropyl alcohol and stirring at 500 rpm for 5 seconds by a homo disper to obtain a dispersion, and measuring the dispersion by a laser diffraction scattering method is preferably 10 µm or greater.

The zinc oxide particles having a surface coated with organosilicon preferably have an apparent density of 0.5 g/mL or greater.

The zinc oxide particles having a surface coated with organosilicon preferably have a silicon content of 0.1 mass% or greater and 0.6 mass% or less.

An embodiment of the present invention relates to a method for producing zinc oxide particles having a surface coated with organosilicon, the method including: a step (1) of preparing a slurry by repulping zinc oxide particles as a raw material in a solvent containing water and an organic solvent; a step (2) of hydrolyzing an organosilicon compound in a solvent containing water and an organic solvent; and a step (3) of subjecting the zinc oxide particles to a surface treatment with a silane compound obtained after the hydrolysis.

The zinc oxide particles are preferably produced by a production method including a step of aging zinc oxide microparticulates in water containing a zinc salt dissolved therein.

For the solvents in the step (1) and the step (2), the organic solvent is preferably contained in an amount of 30 mass% or greater with respect to a total amount of the solvent.

The organic solvent preferably contains at least one selected from the group consisting of methanol, ethanol, and propanol.

The step (2) is preferably performed in the solvent having a pH adjusted to 3.5 to 4.5.

An embodiment of the present invention is a cosmetic preparation containing the zinc oxide particles having a surface coated with organosilicon described above.

An embodiment of the present invention is a dispersion containing the zinc oxide particles having a surface coated with organosilicon described above.

An embodiment of the present invention is a heat dissipating filler containing the zinc oxide particles having a surface coated with organosilicon described above.

An embodiment of the present invention is a resin composition containing the zinc oxide particles having a surface coated with organosilicon described above.

### Advantageous Effects of Invention

The zinc oxide particles having a surface coated with organosilicon of an embodiment of the present invention are adequately treated with a water-repellent treatment and can be suitably used for a cosmetic preparation, a heat dissipating filler, and the like. Furthermore, since the zinc oxide particles have high dispersibility, the zinc oxide particles can be easily dispersed with a weak impact at the time of use.

### Brief Description of Drawings

FIG. 1 is transmission electron micrographs of zinc oxide particles of Example 1 and Comparative Example 4.
FIG. 2 is a chart showing an X-ray diffraction spectrum of zinc oxide particles of Example 1.
FIG. 3 is schematic views illustrating a measurement method of a primary particle size of zinc oxide particles having a surface coated with organosilicon of an embodiment of the present invention.

### Description of Embodiments

Embodiments of the present invention will be described in detail below.

The zinc oxide particles having a surface coated with organosilicon of an embodiment of the present invention are produced by subjecting zinc oxide particles having excellent dispersibility to an organosilicon compound treatment. That is, by subjecting zinc oxide particles dispersed in a state close to a primary particle state in a solvent to an organosilicon compound treatment, a uniform organosilicon coating layer is formed on each particle. Since the obtained organosilicon-coated zinc oxide particles also have excellent dispersibility, when blended in a cosmetic preparation or the like, the organosilicon-coated zinc oxide particles can be dispersed by a weak impact. Meanwhile, when no impact is applied, aggregated particles can be formed by an appropriate cohesive force. Thus, handleability is excellent.

It is conceived that the zinc oxide particles having a surface coated with organosilicon have excellent dispersibility and are dispersed to a state close to a primary particle state in the solvent. Specific indicators includes a primary particle size of 0.1 µm or less and a median size of 0.15 µm or less measured based on a transmission electron micrograph. The median size here corresponds to a median size 2 described below.

Specifically, the primary particle size in an embodiment of the present invention is a particle size (µm) defined as a unidirectional particle size (distance between two parallel lines that are in one direction and that are sandwiching a particle; for particles with all shapes in an image, measurement was performed for one direction) in a magnified field of view of 2000 to 50000 times of an image taken by a transmission electron microscope JEM-1200 EX II (available from JEOL, Ltd.), and determined by measuring unidirectional particle sizes for 250 primary particles in a TEM image, and calculating an average value of the cumulative distribution thereof.

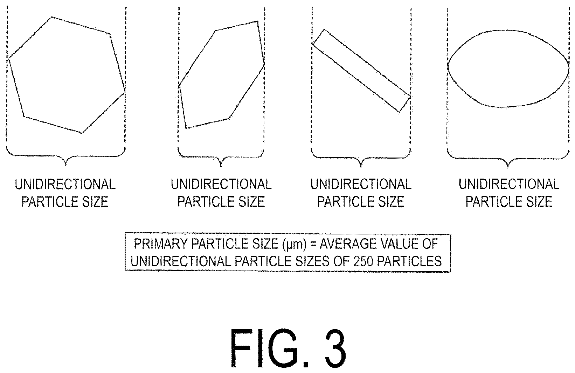

For the measurement method of the primary particle size, FIG. 3 is attached.

The primary particle size is more preferably 0.005 µm or greater and less than 0.1 µm.

Furthermore, specifically, the median size is measured by the following method.

0.4 g of the zinc oxide particles having a surface coated with organosilicon are added to 200 mL of isopropyl alcohol, ultrasonic wave with an oscillation amplitude of 100% is applied thereto at a normal temperature for 30 seconds by using an ultrasonic homogenizer US-600E (available from Nihonseiki Kaisha Ltd.; rated output: 600 W) to prepare a slurry, and the median size, based on volume, is measured by using a laser diffraction/scattering particle size distribution analyzer LA-960S (available from HORIBA, Ltd.).

Furthermore, since the zinc oxide particles having a surface coated with organosilicon of an embodiment of the present invention have significantly high dispersibility, one of the characteristics thereof is that the zinc oxide particles are dispersed adequately by application of only a weak impact by ultrasonic wave.

The median size is more preferably 0.13 µm or less.

In the zinc oxide particles having a surface coated with organosilicon of an embodiment of the present invention, a ratio of the median size to the primary particle size (median size/primary particle size) is preferably 4 or less. The ratio of the median size to the primary particle size is a value serving as an indicator for degree of independency of the primary particles constituting powder, and a value closer to 1 indicates that primary particles are present more independently and not as aggregated particles. The ratio of the median size to the primary particle size is more preferably 3 or less. The ratio is even more preferably 2.5 or less. Note that the median size here corresponds to a median size 2 described below.

Although the zinc oxide particles having a surface coated with organosilicon of an embodiment of the present invention have dispersibility, in a case where dispersing treatment is not performed, the zinc oxide particles are present in a properly aggregated state. Specifically, a median size, based on a volume, determined by charging the powder in isopropyl alcohol and stirring at 500 rpm for 5 seconds by a homo disper to obtain a slurry, and measuring the slurry by a laser diffraction scattering method is preferably 10 µm or greater. That is, the zinc oxide particles form loosely aggregated particles unless a dispersing treatment is performed by a stirrer, ultrasonic wave, or the like, and the zinc oxide particles disperse to a state close to a primary particle state when the aggregation state is loosened upon application of impact.

In the present description, the median size in a state after stirring at 500 rpm for 5 seconds by the homo disper described above is referred to as a median size 1, and the median size in a state after ultrasonic wave dispersion for 30 seconds as described above is referred to as a median size 2. The median size 1 is more preferably 13 µm or greater. Further, the median size 1 is preferably 70 µm or less.

The silane compound coating amount of the zinc oxide particles having a surface coated with organosilicon of an embodiment of the present invention in terms of silicon amount is preferably 0.1 mass% or greater and 0.6 mass% or less. When the amount is less than 0.1 mass%, sufficient water-repellent effect may not be exhibited. When the amount is greater than 0.6 mass%, the purity of the zinc oxide decreases, and the performance may deteriorate.

The silane compound coating amount is a value of silicon measured by subjecting zinc oxide particles having a surface coated with organosilicon which were formed by a pressing machine to semiquantitative analysis by the FP method using an X-ray fluorescence spectrometer (PRIMUS II, available from Rigaku Corporation).

Note that, in an embodiment of the present invention, the value of silicon obtained by the measurement method described above is a value including silicon in the organosilicon compound coating the surface of the zinc oxide particles and silicon in a free organosilicon compound that is not in the coating. However, the free organosilicon compound that is not in the coating usually flows out if a step such as washing or heating is included, and it is conceived that the amount of the silicon in the free organosilicon compound that is not in the coating is a very small amount. Thus, there is no problem considering that the value obtained by the measurement method described above is substantially the same as the amount of silicon in the organosilicon compound coating the zinc oxide particles.

The zinc oxide particles having a surface coated with organosilicon of an embodiment of the present invention preferably have an apparent density of 0.5 g/mL or greater. When the apparent density is 0.5 g/mL or greater, the volume of the powder becomes smaller, which is preferred from the viewpoints of reducing amount of generated dust, facilitating charging into a packaging container, and enabling transporting a large amount of the powder during transport. Furthermore, it is preferred from the viewpoint of facilitating handling of the powder, for example, the zinc oxide particles can be mixed in a container having a smaller volume when mixed with another component. The apparent density is more preferably 0.6 g/mL or greater. A higher apparent density indicates a smaller volume of powder.

The apparent density can be measured by the method described in Examples described below.

The zinc oxide particles having a surface coated with organosilicon of an embodiment of the present invention preferably have a BET specific surface area of 25 m²/g or less. When the BET specific surface area is greater than 25 m²/g, the particle size is smaller, and aggregation tends to occur.

The BET specific surface area can be measured by the method described in Examples described below.

In a case where coating films produced by using the zinc oxide particles having a surface coated with organosilicon after 5 minutes of dispersing and a coating film after 90 minutes of dispersing by a paint conditioner (available from Red Devil) are each measured by a spectrophotometer V-770 (available from JASCO Corporation), the zinc oxide particles having a surface coated with organosilicon of an embodiment of the present invention preferably have a total light transmittance at a wavelength of 310 nm of 30% or less, a total light transmittance at a wavelength of 350 nm of 30% or less, a parallel light transmittance at a wavelength of 500 nm of 70% or greater, and a parallel light transmittance at a wavelength of 700 nm of 80% or greater.

A smaller value of the total light transmittance at the wavelength of 310 nm indicates a higher ultraviolet blocking effect against ultraviolet light having a wavelength of UVB, and a smaller value of the total light transmittance at the wavelength of 350 nm indicates a higher ultraviolet blocking effect against ultraviolet light having a wavelength of UVA. Furthermore, larger values of the parallel light transmittance at the wavelength of 500 nm and the parallel light transmittance at the wavelength of 700 nm indicate higher visible light transmittance. That is, the exhibition of the total light transmittance and the parallel light transmittance in these ranges described above is preferred because excellent ultraviolet blocking performance and transmittance are achieved. A higher performance of a coating film after 5 minutes of dispersion indicates a higher dispersibility. A higher performance of a coating film after 90 minutes of dispersion indicates a higher performance when dispersion is performed sufficiently. The total light transmittance and the parallel light transmittance can be measured by the method described in Examples.

The shape of the zinc oxide particles having a surface coated with organosilicon of an embodiment of the present invention is not particularly limited and is preferably a plate shape from the viewpoint of achieving excellent texture at the time of use and excellent blocking performance against UV and the like.

The method for producing the zinc oxide particles having a surface coated with organosilicon of an embodiment of the present invention are not particularly limited and an example thereof may be a method for producing zinc oxide particles having a surface coated with organosilicon, the method including: a step (1) of preparing a slurry by repulping zinc oxide particles as a raw material in a solvent containing water and an organic solvent; a step (2) of hydrolyzing an organosilicon compound in a solvent containing water and an organic solvent; and a step (3) of subjecting the zinc oxide particles to a surface treatment with a silane compound obtained after the hydrolysis.

Such a production method is also an embodiment of the present invention.

The step (1) is a step of preparing a slurry by repulping zinc oxide particles as a raw material in a solvent containing water and an organic solvent. In the production method of an embodiment of the present invention, preparation of the slurry of the zinc oxide particles in the step (1) is an important step. That is, the step (1) is characterized by formation of organosilicon coating in a dispersed slurry state and not in a dried state in which the zinc oxide particles are aggregated.

Note that the zinc oxide particles used as raw materials in the step (1) are preferably used for slurry preparation as are as the zinc oxide particles that underwent a synthesis reaction in an aqueous medium and that underwent no drying step. That is, preferably, no step of drying the particles is included between the production step of the particles to the step of organosilicon coating treatment. Consequently, aggregation of the particles is suppressed, and surface treatment is not performed for aggregated particles, and thus excellent dispersibility as described above can be achieved.

By this step (1), each one of the zinc oxide particles is surface-treated, thus an excessive organosilicon coating amount is not required, and water repellency can be efficiently imparted.

In the step (1), for example, the slurry of the zinc oxide particles preferably contains, but is not limited to, from 10 to 1000 g/L of zinc oxide particles.

The preparation method of the slurry is not particularly limited. For example, the preparation method may include filtering the zinc oxide particles obtained by the production step of the zinc oxide particles, then adding the zinc oxide particles to water without drying the zinc oxide particles, dispersing the zinc oxide particles at 5 to 30°C for 10 to 30 minutes, and thus forming a uniform slurry containing a zinc oxide particle concentration of 10 to 1000 g/L.

The organic solvent is not particularly limited, and those described below can be used.

The step (2) is a step (2) of hydrolyzing an organosilicon compound in a solvent containing water and an organic solvent. The organosilicon compound is not particularly limited as long as the organosilicon compound is a compound that generates a silanolic hydroxy group by hydrolysis. Examples thereof include methyltriethoxysilane, methyltrimethoxysilane, methyltrichlorosilane, dimethyldiethoxysilane, dimethyldimethoxysilane, dimethyldichlorosilane, phenyltriethoxysilane, phenyltrimethoxysilane, phenyltrichlorosilane, hexyltriethoxysilane, hexyltrimethoxysilane, hexyltrichlorosilane, octyltriethoxysilane, octyltrimethoxysilane, octyltrichlorosilane, decyltriethoxysilane, decyltrimethoxysilane, decyltrichlorosilane, tetraethoxysilane, trimethylchlorosilane, hexamethyldisilazane, hexamethyldisiloxane, perfluoroalkyltrimethoxysilane; organopolysiloxanes, such as methylhydrogenpolysiloxane and dimethylpolysiloxane; and silane coupling agents, such as triethoxyvinylsilane and diphenyldimethoxysilane. One type or a combination of two or more types of the organosilicon compounds may be used.

The organic solvent used in the step (2) is not particularly limited as long as the organic solvent allows hydrolysis of the organosilicon compound and is preferably at least one selected from the group consisting of methanol, ethanol, and propanol. The use of such lower alcohols facilitates hydrolysis. The organic solvent is more preferably ethanol.

The step (2) is preferably performed in a condition at a pH of 3.5 to 4.5. When the step (2) is performed in a condition at a pH of 3.5 to 4.5, hydrolysis of the organosilicon compound can be proceeded in a short period of time, and self-condensation of the silane compound obtained by the hydrolysis can be effectively suppressed. The pH is more preferably from 3.8 to 4.2. The pH is only required to be appropriately adjusted by using an acid or alkali.

The step (2) is preferably performed under stirring. By the stirring, the organosilicon compound can be hydrolyzed more adequately.

The time for performing the hydrolysis step is not particularly limited and is preferably 30 minutes or longer. Furthermore, the temperature at which the hydrolysis is performed is not particularly limited and is preferably 40°C or higher. Under such conditions, the self-condensation of the silane compound can be suppressed while the hydrolysis of the organosilicon compound is adequately proceeded.

The stirring means in the step (2) is not particularly limited, and examples thereof include stirring by an impeller, agitation by shaking, stirring by a mixer, and stirring by a stirrer.

The step (3) is a step of subjecting the zinc oxide particles to a surface treatment with the silane compound obtained after the hydrolysis.

In the surface treatment step, stirring is preferably performed after the silane compound obtained after the hydrolysis is added to the slurry of the zinc oxide particles obtained in the step (1). By the stirring, reaction of a silanol group of the hydrolyzed silane compound can be adequately proceeded, and the surface treatment can be more efficiently performed.

The time for stirring is not particularly limited and is preferably 60 minutes or longer. Examples of the stirring means include a blender and a bead mill.

The temperature at which the surface treatment step is performed is not particularly limited and is preferably performed at 30 to 100°C.

The step (3) is a step of adding the silane compound solution after the hydrolysis, and the addition is preferably performed in such a manner that a charged amount of the silane compound is 1.0 mass% or greater with respect to the amount of the zinc oxide particles. When the charged amount is less than 1.0 mass%, water repellency may be insufficient. The charged amount is more preferably 1.5 mass% or greater. Furthermore, the charged amount is preferably 10 mass% or less, more preferably 8 mass% or less.

In this manner, after the surface treatment step is performed, the obtained slurry is aged for 1 hour to several hours while being stirred, and thus an organosilicon coating is formed on a surface of the zinc oxide particle.

In the aging, a small amount of another component may be added in a range that does not impair the effect of the present invention. For example, a dispersing agent or the like may be added.

The aging is preferably performed at 45 to 110°C. In particular, the aging time may be from 0.5 to 24 hours. The particle size can be adjusted by conditions such as the aging temperature, aging time, and zinc oxide microparticulate concentration, these conditions may be appropriately set based on the target zinc oxide particles.

The zinc oxide particles having a surface coated with organosilicon obtained as described above may, as necessary, undergo post-treatment such as filtering, water-washing, or drying.

For example, the slurry of the organosilicon-coated zinc oxide particles may be filtered to separate and wash the zinc oxide particles having a surface coated with organosilicon with water, then the zinc oxide particles having a surface coated with organosilicon are typically heated at a temperature in a range of 80 to 150°C and dried, and thus the zinc oxide particles having a surface coated with organosilicon can be obtained as dried powder. Note that the zinc oxide particles having a surface coated with organosilicon of an embodiment of the present invention preferably undergo no firing step, such as a step of heating at 300°C or higher, after the surface coating. By such zinc oxide particles having a surface coated with organosilicon, the effect of an embodiment of the present invention described above can be suitably achieved.

The zinc oxide particles as the raw material are not particularly limited; however, use of zinc oxide particles having a higher dispersibility is preferred because the dispersibility of the resulting zinc oxide particles having a surface coated with organosilicon is also increased. The shape of the zinc oxide particle as the raw material is not particularly limited and is preferably a plate shape from the viewpoint of achieving excellent texture at the time of use of the resulting zinc oxide particles having a surface coated with organosilicon and excellent blocking performance against UV and the like. For example, zinc oxide particles having a primary particle size of less than 0.1 µm are preferred, zinc oxide particles having an aspect ratio of less than 2.5 are preferred, and zinc oxide particles having an oil absorption amount/BET specific surface area of 1.5 mL/100 m² or less are preferred. The oil absorption amount/BET specific surface area (mL/100 m²) is a value determined by dividing the value of the oil absorption amount (mL/100 g) by the value of the BET specific surface area (m²/g). A smaller value of the oil absorption amount/BET specific surface area indicates a lower oil absorption amount per unit area of the particle surface, less aggregation of the particles, and high independency and dispersibility of the particles. Examples of such raw material zinc oxide particles include zinc oxide particles obtained by a production method including a step of aging zinc oxide microparticulates having a primary particle size of 0.005 µm or greater and 0.05 µm or less in water, in which zinc salt is dissolved. Specifically, for example, zinc oxide particles described in Patent Document 1 are preferably used.

Patent Document 1 describes a method for obtaining zinc oxide particles having excellent dispersibility by a production method including a step of aging zinc oxide microparticulates in water containing a zinc salt dissolved therein. After the zinc oxide particles are produced in the water by the method described above, the zinc oxide particles are preferably filtered and surface-treated by the method described above without being dried. As a result, the surface treatment can be performed for the zinc oxide particles, which are less aggregated, and the object of the present invention can be suitably achieved.

By the production method described in Patent Document 1, zinc oxide particles having a primary particle size of less than 0.1 µm, an aspect ratio of less than 2.5, and an oil absorption amount/BET specific surface area of 1.5 mL/100 m² or less can be obtained. Such zinc oxide particles are particularly preferably used as the raw material.

For the zinc oxide particles having a surface coated with organosilicon of an embodiment of the present invention, another surface treatment may be further performed as necessary.

The surface treatment is not particularly limited, and examples thereof include a known treatment method such as an inorganic surface treatment for forming an inorganic oxide layer, such as a silica layer, an alumina layer, a zirconia layer, or a titania layer, or other various surface treatments. Furthermore, a plurality of types of surface treatments may be performed successively.

More specific examples of the surface treatment include a surface treatment by a surface treatment agent selected from the group consisting of an organoaluminum compound, an organotitanium compound, a higher fatty acid, a higher fatty acid ester, a metallic soap, a polyhydric alcohol, and an alkanolamine. For such a surface treatment agent, the treatment amount can be appropriately set based on the particle size of the zinc oxide particle.

The zinc oxide particles having a surface coated with organosilicon of an embodiment of the present invention may be a dispersion in which the zinc oxide particles are dispersed in water or an oily agent.

Such a dispersion is also an aspect of the present invention.

The use of the zinc oxide particles having a surface coated with organosilicon of an embodiment of the present invention is not particularly limited and, for example, can be suitably used for a raw material of a cosmetic preparation or for a heat dissipating filler. Such a cosmetic preparation and a heat dissipating filler are also aspects of the present invention.

The cosmetic preparation containing the zinc oxide particles having a surface coated with organosilicon of an embodiment of the present invention achieve excellent dispersibility and stability and good handleability.

Examples of the cosmetic preparation of an embodiment of the present invention include foundations, makeup bases, eye shadows, blushers, mascaras, lipsticks, sunscreen agents, and the like. The cosmetic preparation of an embodiment of the present invention may be in any form selected from an oil-based cosmetic, a water-based cosmetic, an O/W cosmetic, or a W/O cosmetic. Among these, the cosmetic preparation can be particularly suitably used in sunscreen agents.

In the cosmetic preparation of an embodiment of the present invention, any aqueous component or oily component that can be used in the field of cosmetic preparations can be used in combination in addition to the components constituting the mixture described above. The aqueous component and oily component described above are not particularly limited. Examples thereof may include those containing components such as oils, surfactants, moisturizers, higher alcohols, sequestrants, natural and synthetic polymers, water-soluble and oil-soluble polymers, ultraviolet blocking agents, various extracts, inorganic and organic pigments, inorganic and organic clay minerals, inorganic and organic pigments that are metallic soap-treated or silicone-treated, coloring materials such as organic dyes, preservatives, antioxidants, dyes, thickeners, pH adjusters, perfumes, cooling-sensation agents, antiperspirants, disinfectants, and skin activators. Specifically, a desired cosmetic can be produced in the usual manner using any one or two or more types of the ingredients listed below. The compounded amounts of these ingredients are not particularly limited as long as the compounded amounts are in a range that does not impair the effect of the present invention.

The oil is not particularly limited. Examples thereof may include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, arachis oil, tea seed oil, kaya oil, rice bran oil, Chinese tung oil, Japanese tung oil, jojoba oil, germ oil, triglycerol, glycerol trioctanoate, glycerol triisopalmitate, cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol ester, POE hydrogenated lanolin alcohol ether, liquid paraffin, ozokerite, pristane, paraffin, ceresin, squalene, vaseline, microcrystalline wax, and the like.

The lipophilic nonionic surfactant described above is not particularly limited. Examples thereof may include sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerin polyglycerin fatty acid esters, such as glycerol mono-cottonseed oil fatty acid, glycerol monoerucate, glycerol sesquioleate, glycerol monostearate, α,α'-glycerol oleate pyroglutamate, and glycerol monostearate malate; propylene glycol fatty acid esters, such as propylene glycol monostearate; hydrogenated castor oil derivatives; and glycerol alkyl ethers.

The hydrophilic nonionic surfactant described above is not particularly limited. Examples thereof may include POE sorbitan fatty acid esters, such as POE sorbitan monostearate, POE sorbitan monooleate, and POE sorbitan tetraoleate; POE sorbitol fatty acid esters, such as POE sorbitol monolaurate, POE sorbitol monooleate, POE sorbitol pentaoleate, and POE sorbitol monostearate; POE glycerin fatty acid esters, such as POE glycerin monostearate, POE glycerin monoisostearate, and POE glycerin triisostearate; POE fatty acid esters, such as POE monooleate, POE distearate, POE monodioleate, and ethylene glycol distearate; POE alkyl ethers, such as POE lauryl ether, POE oleyl ether, POE stearyl ether, POE behenyl ether, POE 2-octyldodecyl ether, and POE cholestanol ether; POE alkyl phenyl ethers, such as POE octyl phenyl ether, POE nonyl phenyl ether, and POE dinonyl phenyl ether; Pluaronic types, such as Pluronic; POE/POP alkyl ethers, such as POE/POP cetyl ether, POE/POP 2-decyltetradecyl ether, POE/POP monobutyl ether, POE/POP hydrogenated lanolin, and POE/POP glycerin ether; tetra-POE/tetra-POP ethylenediamine condensation products, such as Tetronic; POE castor oil hydrogenated castor oil derivatives, such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester, and POE hydrogenated castor oil maleic acid; POE beeswax/lanolin derivatives, such as POE sorbitol beeswax; alkanolamides, such as coconut oil fatty acid diethanolamide, lauric acid monoethanolamide, and fatty acid isopropanol amide; POE propylene glycol fatty acid esters; POE alkylamines; POE fatty acid amides; sucrose fatty acid esters; POE nonylphenyl formaldehyde condensation products; alkyl ethoxy dimethylamine oxides; and trioleyl phosphoric acid.

Other surfactants may be compounded in a range that does not cause any problems in stability and skin irritation. Examples thereof include anionic surfactants such as fatty acid soaps, higher-alkyl sulfuric ester salts, POE triethanolamine lauryl sulfate, and alkyl ether sulfuric ester salts; cationic surfactants such as alkyl trimethylammonium salts, alkyl pyridinium salts, alkyl quaternary ammonium salts, alkyl dimethylbenzyl ammonium salts, POE alkylamines, alkylamine salts, and polyamine fatty acid derivatives; and amphoteric surfactants such as imidazoline amphoteric surfactants and betaine surfactants.

The moisturizer is not particularly limited. Examples thereof may include xylitol, sorbitol, maltitol, chondroitin sulfate, hyaluronic acid, mucoitinsulfuric acid, caronic acid, atelocollagen, cholesteryl-12-hydroxystearate, sodium lactate, bile salts, dl-pyrrolidone carboxylate, short-chain soluble collagens, diglycerol (EO) PO adducts, Rosa roxburghii extract, yarrow extract, melilot extract, and the like.

The higher alcohol described above is not particularly limited. Examples thereof may include linear alcohols such as lauryl alcohol, cetyl alcohol, stearyl alcohol, behenyl alcohol, myristyl alcohol, oleyl alcohol, and cetostearyl alcohol; branched alcohols such as monostearyl glycerol ether (batyl alcohol), 2-decyltetradecynol, lanolin alcohol, cholesterol, phytosterol, hexyldodecanol, isostearyl alcohol, and octyldodecanol; and the like.

The sequestrant is not particularly limited. Examples thereof may include 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, edetic acid, and the like.

The natural water-soluble polymer is not particularly limited. Examples thereof may include plant-derived polymers such as gum arabic, tragacanth gum, galactan, guar gum, carob gum, karaya gum, carrageenan, pectin, agar, quince seed (quince), algal colloid (algal extract), starch (rice, corn, potato, wheat), and glycyrrhizinic acid; microorganism-derived polymers such as xanthan gum, dextran, succinoglucan, and pullulan; and animal-derived polymers such as collagen, casein, albumin, and gelatin.

The semisynthetic water-soluble polymer is not particularly limited. Examples thereof may include starch polymers such as carboxymethyl starch and methyl hydroxypropyl starch; cellulose polymers such as methyl cellulose, nitro cellulose, ethyl cellulose, methyl hydroxypropyl cellulose, hydroxyethyl cellulose, cellulose sodium sulfate, hydroxypropyl cellulose, sodium carboxymethylcellulose (CMC), crystalline cellulose, and cellulose powder; alginate polymers such as sodium alginate and propylene glycol alginate; and the like.

The synthetic water-soluble polymer is not particularly limited. Examples thereof may include vinyl polymers such as polyvinyl alcohol, polyvinyl methyl ether, and polyvinyl pyrrolidone; polyoxyethylene polymers such as polyethylene glycol 20000, polyethylene glycol 40000, and polyethylene glycol 60000; copolymers such as polyoxyethylene-polyoxypropylene copolymers; acrylic polymers such as sodium polyacrylate, polyethylacrylate, and polyacrylamide; polyethyleneimine; cationic polymers; and the like.

The inorganic water-soluble polymer is not particularly limited. Examples thereof may include bentonite, magnesium aluminum silicate (Veegum), laponite, hectorite, silicic anhydride, and the like.

The ultraviolet blocking agent is not particularly limited. Examples thereof may include benzoic acid-based ultraviolet blocking agents such as paraaminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, and N,N-dimethyl PABA butyl ester; anthranilic acid-based ultraviolet blocking agents such as homomenthyl-N-acetyl anthranilate; salicylic acid-based ultraviolet blocking agents such as amyl salicylate, menthyl salicylate, homomenthyl salicylate, octyl salicylate, phenyl salicylate, benzyl salicylate, and p-isopropanol phenyl salicylate; cinnamic acid-based ultraviolet blocking agents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glycerylmono-2-ethylhexanoyl-diparamethoxy cinnamate; benzophenone-based ultraviolet blocking agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor, 3-benzylidene-d,1-camphor, urocanic acid, urocanic acid ethyl ester, 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenyl)benzotriazole, dibenzalazine, dianisoylmethane, 4-methoxy-4'-t-butyldibenzoylmethane, 5-(3,3-dimethyl-2-norbornylidene)-3-pentan-2-one, and the like.

Other chemical components are not particularly limited, and examples thereof include vitamins, such as vitamin A oil, retinol, retinol palmitate, inositol, pyridoxine hydrochloride, benzyl nicotinate, nicotinamide, DL-α-tocopherol nicotinate, magnesium ascorbyl phosphate, 2-O-α-D-glucopyranosyl-L-ascorbic acid, vitamin D2 (ergocalciferol), DL-α-tocopherol, DL-α-tocopherol acetate, pantothenic acid, and biotin; hormones, such as estradiol and ethynyl estradiol; amino acids, such as arginine, aspartic acid, cystine, cysteine, methionine, serine, leucine, and tryptophan; anti-inflammatory agents, such as allantoin and azulene; whitening agents, such as arbutin; astringents, such as tannic acid; refrigerants, such as L-menthol and camphor, sulfur, lysozyme chloride, and pyridoxine chloride.

Various kinds of extracts are not particularly limited. Examples thereof may include Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of the various kinds of powders described above may include bright coloring pigments, such as red oxide, yellow iron oxide, black iron oxide, mica titanium, iron oxide-coated mica titanium, and titanium oxide-coated glass flakes, inorganic powders, such as those of mica, talc, kaolin, sericite, titanium dioxide, and silica, organic powders, such as polyethylene powder, nylon powder, crosslinked polystyrene, cellulose powder, and silicone powder, and the like. Preferably, for enhancing sensory characteristics and makeup retainability, part or all of the powder component may be subjected to a hydrophobization treatment by a publicly known method using a substance such as silicones, a fluorine compound, a metallic soap, an oily agent, or an acyl glutamic acid salt, for use. Other zinc oxide particles that do not fall under the present invention may be mixed and used.

The zinc oxide particles having a surface coated with organosilicon silica of an embodiment of the present invention can be used as a heat dissipating filler.

In a case where the zinc oxide particles having a surface coated with organosilicon of an embodiment of the present invention are used as a heat dissipating filler, a combined use with a heat dissipating filler having a particle size greater than that of the zinc oxide particles having a surface coated with organosilicon of an embodiment of the present invention is preferred. The heat dissipating filler that can be used in combination is not particularly limited, and examples thereof include metal oxides such as magnesium oxide, titanium oxide, and aluminum oxide; aluminum nitride, boron nitride, silicon carbide, silicon nitride, titanium nitride, metallic silicon, and diamond. Furthermore, a combination with zinc oxide other than the zinc oxide particles having a surface coated with organosilicon can be also used. The heat dissipating filler used in combination may have any shape such as a spherical shape, a needle shape, a rod shape, or a plate shape.

In a case where the zinc oxide particles having a surface coated with organosilicon of an embodiment of the present invention are used in combination with another heat dissipating filler, the heat dissipating filler used in combination preferably has an average particle size of 1 to 100 µm. Combining with such a heat dissipating filler having a large particle size is preferred from the viewpoints of charging the heat dissipating filler of an embodiment of the present invention in gaps and increasing filling percentage.

In a case where the zinc oxide particles having a surface coated with organosilicon of an embodiment of the present invention are used in combination with an additional heat dissipating filler, from 10 to 90 vol.% of the zinc oxide particles having a surface coated with organosilicon of an embodiment of the present invention are preferably contained with respect to the total amount of the heat dissipating fillers. Such a proportion can increase the filling percentage.

In a case where the zinc oxide particles having a surface coated with organosilicon are used as a heat dissipating filler, the zinc oxide particles having a surface coated with organosilicon can be used as a heat dissipating resin composition obtained by mixing with a resin. The resin used in this case may be a thermoplastic resin or a thermosetting resin, and examples thereof include resins such as an epoxy resin, a phenolic resin, a polyphenylene sulfide (PPS) resin, a polyester-based resin, polyamide, polyimide, polystyrene, polyethylene, polypropylene, polyvinyl chloride, polyvinylidene chloride, a fluororesin, a poly(methyl methacrylate), ethylene-ethyl acrylate copolymer (EEA) resin, polycarbonate, polyurethane, polyacetal, polyphenylene ether, polyetherimide, an acrylonitrile-butadiene-styrene copolymer (ABS) resin, a liquid crystal resin (LCP), a silicone resin, and an acrylic resin.

The heat dissipating resin composition of an embodiment of the present invention may be (1) a resin composition for thermoforming, the resin composition being obtained by kneading a thermoplastic resin and the zinc oxide particles having a surface coated with organosilicon in a molten state; (2) a resin composition obtained by heating and curing after a thermosetting resin and the zinc oxide particles having a surface coated with organosilicon are kneaded; or (3) a resin composition for a coating material, the resin composition being obtained by dispersing the zinc oxide particles having a surface coated with organosilicon in a resin solution or dispersion.

In a case where the heat dissipating resin composition of an embodiment of the present invention is a resin composition for thermoforming, resin components can be freely selected based on the use. For example, in a case where close adhesion to a heat source and a heat sink is performed, a resin having a high adhesion and a low hardness, such as a silicone resin or an acrylic resin, may be selected.

In a case where the heat dissipating resin composition of an embodiment of the present invention is a resin composition for a coating material, the resin may be curable or non-curable. The coating material may be a solvent-based coating material containing an organic solvent or may be a water-based coating material in which the resin is dissolved or dispersed in water.

In a case where the zinc oxide particles having a surface coated with organosilicon are used as a heat dissipating filler, the zinc oxide particles having a surface coated with organosilicon can be used as a heat dissipating grease obtained by mixing with a base oil containing a mineral oil or a synthetic oil. In a case of use as such a heat dissipating grease, as the synthetic oil, for example, α-olefin, diester, polyol ester, trimellitate, polyphenyl ether, and alkyl phenyl ether can be used. Furthermore, the zinc oxide particles having a surface coated with organosilicon can be used as a heat dissipating grease obtained by mixing with a silicone oil.

The zinc oxide particles having a surface coated with organosilicon of an embodiment of the present invention are used as a heat dissipating filler, an additional component can be used in combination. Examples of such an additional component that can be used in combination include a resin and a surfactant.

In addition to the cosmetic preparation and the heat dissipating filler described above, the zinc oxide particles having a surface coated with organosilicon of an embodiment of the present invention can be used in fields such as a vulcanization accelerator for a rubber, a pigment for a coating material and ink, an electronic component such as a ferrite and a varistor, and a pharmaceutical product.

### Examples

The present invention will be described hereinafter with reference to examples, but the present invention is not limited to these examples. In Examples, a blending proportion described by "%" refers to "mass%" unless otherwise noted.

### Production Example of raw material zinc oxide cake

80 g of FINEX-50 (available from Sakai Chemical Industry Co., Ltd.; primary particle size: 0.020 µm) was repulped in 1200 mL of a zinc acetate aqueous solution having a zinc acetate concentration of 0.135 mol/L, and thus a slurry was obtained. Subsequently, the temperature of the slurry was increased to 70°C over 42 minutes while the slurry was agitated, and then the slurry was aged at 70°C for 2 hours while being agitated. After the aging, filtration and water washing were performed. Subsequently, the obtained solid material was repulped in 3 L of water to form a slurry, the temperature of the slurry was increased to 95°C while the slurry was agitated, and heating and washing was performed at 95°C for 60 minutes while the slurry was agitated. After the heating and washing, filtering and water washing were performed, and thus a zinc oxide cake was obtained.

### Example 1

The cake obtained in Production Example was repulped in an ethanol aqueous solution obtained by mixing 200 mL of ethanol and 65 mL of pure water in such a manner that the ethanol concentration was 44%, and thus a 44% ethanol aqueous solution slurry was obtained. The pH of 20 mL of 44% ethanol aqueous solution was adjusted to pH 4 by using acetic acid, 2.0 g (2.5% with respect to the amount of zinc oxide) of octyltriethoxysilane (KBE-3083, available from Shin-Etsu Chemical Co., Ltd.) was added and agitated at 80°C for 2 hours, and thus an octyltriethoxysilane hydrolysis solution was obtained. The temperature of the 44% ethanol aqueous solution slurry was increased to 50°C, and the octylsilane hydrolysis solution was added thereto and aged for 2 hours while the temperature of 50°C was maintained. After the aging, filtering and water washing were performed and then drying at 120°C for 16 hours was performed, and thus octyltriethoxysilane-coated zinc oxide particles having a primary particle size of 0.061 µm were obtained. The size and form of the obtained particles were observed by using a transmission electron microscope (TEM, JEM-1200EX II, available from JEOL, Ltd.). The evaluation results of physical properties of the obtained particles and physical properties of the coating film are shown in Table 1.

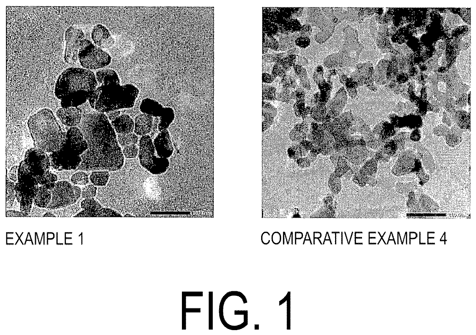

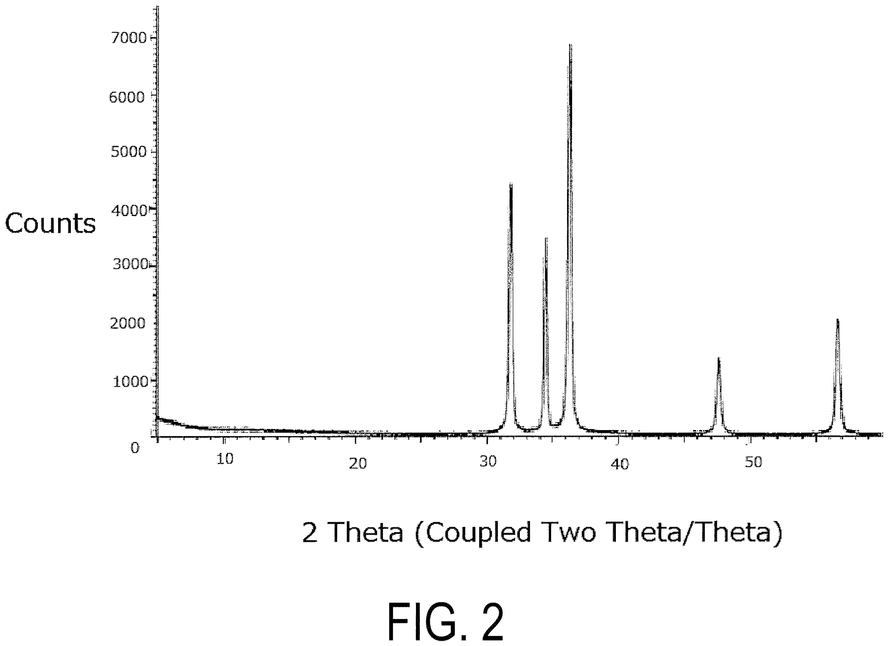

Furthermore, an electron micrograph of the obtained particles is shown in FIG. 1, and an X-ray diffraction spectrum is shown in FIG. 2.

### Example 2

Octyltriethoxysilane-coated zinc oxide particles were obtained in the same manner as in Example 1 except for producing the octyltriethoxysilane hydrolysis solution by adjusting the pH of 8 mL of the 44% ethanol aqueous solution to pH 4 by using acetic acid, adding 0.8 g (1% with respect to the amount of zinc oxide) of octyltriethoxysilane, and agitating at 80°C for 2 hours.

### Example 3

Octyltriethoxysilane-coated zinc oxide particles were obtained in the same manner as in Example 1 except for producing the octyltriethoxysilane hydrolysis solution by adjusting the pH of 24 mL of the 44% ethanol aqueous solution to pH 4 by using acetic acid, adding 2.4 g (3% with respect to the amount of zinc oxide) of octyltriethoxysilane, and agitating at 80°C for 2 hours.

### Example 4

Octyltriethoxysilane-coated zinc oxide particles were obtained in the same manner as in Example 3 except for repulping the cake obtained in Production Example in an ethanol aqueous solution obtained by mixing 240 mL of ethanol and 25 mL of pure water in such a manner that the ethanol concentration was 55%, to form a 55% ethanol aqueous solution slurry.

### Example 5

Octyltriethoxysilane-coated zinc oxide particles were obtained in the same manner as in Example 1 except for using phenyltrimethoxysilane (KBM-103, available from Shin-Etsu Chemical Co., Ltd.) in place of octyltriethoxysilane.

### Example 6

Octyltriethoxysilane-coated zinc oxide particles were obtained in the same manner as in Example 1 except for using phenyltriethoxysilane (KBE-103, available from Shin-Etsu Chemical Co., Ltd.) in place of octyltriethoxysilane.

### Example 7

Octyltriethoxysilane-coated zinc oxide particles were obtained in the same manner as in Example 1 except for producing the octyltriethoxysilane hydrolysis solution by adjusting the pH of 56 mL of the 44% ethanol aqueous solution to pH 4 by using acetic acid, adding 5.6 g (7% with respect to the amount of zinc oxide) of octyltriethoxy, and agitating at 80°C for 2 hours.

### Comparative Example 1

Octyltriethoxysilane-coated zinc oxide particles were obtained in the same manner as in Example 1 except for adding octyltriethoxysilane without hydrolysis.

### Comparative Example 2

The same procedure as in Production Example was performed until heating and washing, filtering, and water washing were performed, the obtained solid material was dried at 120°C for 16 hours by using a dryer, and thus a zinc oxide dried material was obtained. The obtained dried material was crushed by a coffee mill, and thus zinc oxide powder was obtained. The obtained zinc oxide powder was repulped in an ethanol aqueous solution obtained by mixing 200 mL of ethanol and 65 mL of pure water in such a manner that the ethanol concentration was 44%, and thus a 44% ethanol aqueous solution slurry was obtained. Thereafter, the same procedure as in Example 3 was performed to obtain octyltriethoxysilane-coated zinc oxide particles.

### Comparative Example 3

The same procedure as in Comparative Example 2 was performed until the zinc oxide powder was obtained. The pH of 24 mL of the 44% ethanol aqueous solution was adjusted to pH 4 by using acetic acid, 2.4 g (3% with respect to the amount of zinc oxide) of octyltriethoxysilane was added and agitated at 80°C for 2 hours, and thus an octyltriethoxysilane hydrolysis solution was obtained. The zinc oxide powder was placed in a plastic bag, then the octyltriethoxysilane hydrolysis solution was added and mixed by spraying using a sprayer, the mixture was subjected to heat treatment at 120°C for 4 hours by using a dryer, and thus octyltriethoxysilane-coated zinc oxide particles were obtained.

### Comparative Example 4

The same procedure as in Comparative Example 3 was performed until the zinc oxide powder was obtained. The obtained zinc oxide powder was placed in a plastic bag, then 2.4 g (3% with respect to the amount of zinc oxide) of octyltriethoxysilane was added and mixed, the mixture was subjected to heat treatment at 120°C for 16 hours by using a dryer, and thus octyltriethoxysilane-coated zinc oxide particles were obtained.

Furthermore, an electron micrograph of the obtained particles is shown in FIG. 1.

### Comparative Example 5

FINEX-30-OTS (available from Sakai Chemical Industry Co., Ltd.; average particle size: 35 nm) was used.

### Comparative Example 6

FINEX-50-OTS (available from Sakai Chemical Industry Co., Ltd.; average particle size: 20 nm) was used.

### Evaluation Method 1: Composition of obtained particles

The X-ray diffraction spectrum of zinc oxide particles of Example 1 in FIG. 2 and the compositions of the obtained particles shown in Tables 1 and 2 are analysis results by an X-ray diffraction analyzer D8 ADVANCE (available from Bruker). From these results, zinc oxide was clearly obtained for each of Examples and Comparative Examples.

### Evaluation Method 2: Primary particle size

The primary particle size in the present description is a particle size (µm) defined as a unidirectional particle size (distance between two parallel lines that are in one direction and that are sandwiching a particle; for particles with all shapes in an image, measurement was performed for one direction) in a magnified field of view of 2000 to 50000 times of a photograph taken by a transmission electron microscope JEM-1200 EX II (available from JEOL, Ltd.; rated output: 600 W), and was determined by measuring unidirectional particle sizes for 250 primary particles in a TEM photograph, and calculating an average value of the cumulative distribution thereof.

### Evaluation Method 3: BET specific surface area

The BET specific surface area (m²/g) was measured by Full Automatic BET Surface Area Analyzer Macsorb Model HM-1200 (available from Mountech Co., Ltd.).

### Evaluation Method 4: Silicon content

The powder formed by a pressing machine was subjected to semiquantitative analysis by the FP method using an X-ray fluorescence spectrometer (PRIMUS II, available from Rigaku Corporation).

### Evaluation Method 5: Median size 1, median size 2

The median size 1 and the median size 2 were measured using a laser diffraction/scattering particle size distribution analyzer LA-960S (available from HORIBA, Ltd.). 0.4 g of the zinc oxide particles of each of Examples and Comparative Examples were added in 200 mL of isopropyl alcohol. For the median size 1, a slurry obtained by agitating at 500 rpm for 5 seconds by using a homo disper was measured. For the median size 2, a slurry obtained by dispersing the slurry by ultrasonic wave for 30 seconds by using an ultrasonic homogenizer US-600E (available from NISSEI Corporation.) was measured. The measurement based on volume was performed with the refractive index of the zinc oxide of each of Examples and Comparative Examples being 2.00 and the refractive index of isopropyl alcohol being 1.378. The median size 1 indicates a size of the particle, and the median size 2 indicates a size of the particle when the particles are weakly dispersed.

### Evaluation Method 6: Water repellency

50 mL of distilled water was charged in a 100 mL beaker, 1.0 g of the zinc oxide particles were floated and stirred 10 times by a spatula, turbidity of water was visually observed, and evaluation was performed based on the following criteria.
O: No turbidity was observed in the water, and the water was transparent
△: Slight turbidity was observed in the water, and the water was semi-transparent
×: White turbidity was observed in the water

### Evaluation Method 7: Apparent density

### Apparent density

The measurement was performed by a method described in JIS K 5101-12-1 "Test methods for pigments - Part 12: Apparent density or apparent specific volume - Section 1: Loose packing method".

### Evaluation Method 8

### Preparation of coating film

2 g of the zinc oxide particles of each of Examples and Comparative Examples, 10 g of varnish (ACRYDIC A-801-P, available from DIC Corporation), 5 g of butyl acetate (Special Grade Reagent, Wako Pure Chemical Industries, Ltd), 5 g of xylene (Guaranteed Reagent, available from Junsei Chemical Co., Ltd.), and 38 g of glass beads (1.5 mm, available from Potters-Ballotini Co., Ltd.) were placed in a mayonnaise jar having a volume of 75 mL and mixed well, and then the jar was fixed to a paint conditioner (available from Red Devil). A coating material 1 was obtained after agitation for 5 minutes, and a coating material 2 was obtained after agitation for 90 minutes. Then, a small amount of each of the produced coating material 1 and coating material 2 was dropped on a slide glass (length: 76 mm; width: 26 mm; thickness: 0.8 to 1.0 mm; available from Matsunami Glass Ind., Ltd.), and a coating film 1 and a coating film 2 were produced by using a bar coater (No. 579 ROD No.6, available from Yasuda Seiki Seisakusho, Ltd.). After the produced coating film 1 and coating film 2 were dried at 20°C for 12 hours, the coating film 1 and the coating film 2 were used for measurements of the total light transmittance 1, total light transmittance 2, parallel light transmittance 1, and parallel light transmittance 2 described below. A higher performance of the coating film 1 indicates a higher dispersibility. A higher performance of the coating film 2 indicates a higher performance when dispersion is performed sufficiently.

### Total light transmittance 1, total light transmittance 2, parallel light transmittance 1, and parallel light transmittance 2

In the present description, the total light transmittance 1 (%), the total light transmittance 2 (%), the parallel light transmittance 1 (%), and the parallel light transmittance 2 (%) are each a value determined by measuring the produced coating film using the Spectrophotometer V-770 (available from JASCO Corporation). Note that the value of the total light transmittance 1 (%) is a value of total light transmittance at the wavelength of 310 nm, the total light transmittance 2 (%) is a value of total light transmittance at the wavelength of 350 nm, the value of the parallel light transmittance 1 (%) is a value of the parallel light transmittance at the wavelength of 500 nm, and the parallel light transmittance 2 (%) is a value of the parallel light transmittance at the wavelength of 700 nm. A smaller value of the total light transmittance 1 (%) indicates a higher ultraviolet blocking effect against ultraviolet light having a wavelength of UVB, and a smaller value of the total light transmittance 2 (%) indicates a higher ultraviolet blocking effect against ultraviolet light having a wavelength of UVA. Furthermore, larger values of the parallel light transmittance 1 (%) and the parallel light transmittance 2 (%) indicate higher visible light transmittance.

**Table 1-11**

| | | Example 1 | Example 2 | Example 3 | Example 4 |
|---|---|---|---|---|---|
| Surface treatment conditions | Drying of raw material zinc oxide cake | No | No | No | No |
| | Organic solvent in solvent | 44% Ethanol | 44% Ethanol | 44% Ethanol | 55% Ethanol |
| | Surface treatment agent | Octyltriethoxysilane | Octyltriethoxysilane | Octyltriethoxysilane | Octyltriethoxysilane |
| | Charged amount of surface treatment agent (with respect to amount of zinc oxide) | 2.5% | 1% | 3% | 3% |
| | Hydrolysis | Yes | Yes | Yes | Yes |
| | Treatment temperature (°C) | 50 | 50 | 50 | 50 |
| | Treatment time (Hr) | 2 | 2 | 2 | 2 |
| Physical properties of particles | Composition of obtained particles | Zinc oxide | Zinc oxide | Zinc oxide | Zinc oxide |
| | Primary particle size (µm) | 0.061 | 0.061 | 0.059 | 0.062 |
| | BET specific surface area (m²/g) | 17.3 | 19.3 | 17.5 | 18.0 |
| | Silicon content | 0.19% | 0.11% | 0.24% | 0.25% |
| | Median size 1 (µm) | 15.22 | 27.58 | 24.10 | 32.75 |
| | Median size 2 (µm) | 0.11 | 0.10 | 0.13 | 0.09 |
| | Median size 2/primary particle size | 1.8 | 1.6 | 2.2 | 1.5 |
| | Water repellency | O | Δ | O | O |
| | Apparent density (g/mL) | 1.17 | 1.21 | 1.24 | 0.91 |
| Physical properties of coating film 1 | Total light transmittance 1 | 20% | 28% | 20% | 15% |
| | Total light transmittance 2 | 19% | 27% | 19% | 15% |
| | Parallel light transmittance 1 | 85% | 82% | 85% | 85% |
| | Parallel light transmittance 2 | 93% | 89% | 94% | 94% |
| Physical properties of coating film 2 | Total light transmittance 1 | 16% | 15% | 15% | 13% |
| | Total light transmittance 2 | 15% | 15% | 15% | 12% |
| | Parallel light transmittance 1 | 90% | 88% | 87% | 89% |
| | Parallel light transmittance 2 | 98% | 96% | 96% | 98% |

**[Table 1-2]**

| | | Example 5 | Example 6 | Example 7 |
|---|---|---|---|---|
| Surface treatment conditions | Drying of raw material zinc oxide cake | No | No | No |
| | Organic solvent in solvent | 44% Ethanol | 44% Ethanol | 44% Ethanol |
| | Surface treatment agent | Phenyltrimethoxysilane | Phenyltriethoxysilane | Octyltriethoxysilane |
| | Charged amount of surface treatment agent (with respect to amount of zinc oxide) | 2.5% | 2.5% | 7% |
| | Hydrolysis | Yes | Yes | Yes |
| | Treatment temperature (°C) | 50 | 50 | 50 |
| | Treatment time (Hr) | 2 | 2 | 2 |
| Physical properties of particles | Composition of obtained particles | Zinc oxide | Zinc oxide | Zinc oxide |
| | Primary particle size (µm) | 0.062 | 0.060 | 0.058 |
| | BET specific surface area (m²/g) | 20.1 | 19.6 | 14.1 |
| | Silicon content | 0.16% | 0.14% | 0.44% |
| | Median size 1 (µm) | 29.66 | 28.76 | 63.09 |
| | Median size 2 (µm) | 0.09 | 0.09 | 0.10 |
| | Median size 2/primary particle size | 1.5 | 1.5 | 1.7 |
| | Water repellency | O | O | O |
| | Apparent density (g/mL) | 1.20 | 1.16 | 0.87 |
| Physical properties of coating film 1 | Total light transmittance 1 | 19% | 18% | 29% |
| | Total light transmittance 2 | 19% | 18% | 25% |
| | Parallel light transmittance 1 | 85% | 85% | 75% |
| | Parallel light transmittance 2 | 93% | 94% | 90% |
| Physical properties of coating film 2 | Total light transmittance 1 | 13% | 13% | 26% |
| | Total light transmittance 2 | 12% | 12% | 25% |
| | Parallel light transmittance 1 | 88% | 87% | 76% |
| | Parallel light transmittance 2 | 97% | 97% | 91% |

**Table 2]**

| | | Comparative Example 1 | Comparative Example 2 | Comparative Example 3 | Comparative Example 4 | Comparative Example 5 | Comparative Example 6 |
|---|---|---|---|---|---|---|---|
| Surface treatment conditions | Drying of raw material zinc oxide cake | No | Yes | Yes | Yes | | |
| | Organic solvent in solvent | 44% Ethanol | 44% Ethanol | No | No | | |
| | Surface treatment agent | | Octyltriethc | oxysilane | | | |
| | Charged amount of surface treatment agent (with respect to amount of zinc oxide) | 2.5% | 3% | 3% | 3% | | |
| | Hydrolysis | No | Yes | Yes | No | | |
| | Treatment temperature (°C) | 50 | 50 | 120 | 120 | | |
| | Treatment time (Hr) | 2 | 2 | 4 | 16 | | |
| Physical properties of particles | Composition of obtained particles | Zinc oxide | Zinc oxide | Zinc oxide | Zinc oxide | Zinc oxide | Zinc oxide |
| | Primary particle size (µm) | 0.059 | 0.061 | 0.063 | 0.064 | 0.044 | 0.034 |
| | BET specific surface area (m²/g) | 16.8 | 17.9 | 18.1 | 19.9 | 27.3 | 51.0 |
| | Silicon content | 0.09% | 0.23% | 0.22% | 0.17% | 0.46% | 0.83% |
| | Median size 1 (µm) | 23.88 | 19.33 | 13.85 | 11.93 | 6.44 | 6.53 |
| | Median size 2 (µm) | 0.17 | 5.18 | 7.39 | 11.06 | 2.75 | 2.54 |
| | Median size 2/primary particle size | 2.9 | 85.0 | 117.3 | 172.8 | 62.5 | 74.7 |
| | Water repellency | × | ○ | ○ | ○ | ○ | ○ |
| | Apparent density (g/mL) | 1.12 | 0.86 | 1.14 | 0.88 | 0.47 | 0.29 |
| Physical properties of coating film 1 | Total light transmittance 1 | 23% | 31% | 52% | 36% | 33% | 29% |
| | Total light transmittance 2 | 22% | 30% | 49% | 28% | 32% | 30% |
| | Parallel light transmittance 1 | 81% | 70% | 57% | 63% | 73% | 68% |
| | Parallel light transmittance 2 | 90% | 77% | 67% | 70% | 77% | 75% |
| Physical properties of coating film 2 | Total light transmittance 1 | 13% | 16% | 24% | 16% | 16% | 21% |
| | Total light transmittance 2 | 13% | 16% | 23% | 15% | 16% | 22% |
| | Parallel light transmittance 1 | 85% | 82% | 54% | 80% | 92% | 90% |
| | Parallel light transmittance 2 | 95% | 92% | 73% | 90% | 96% | 95% |

From Tables 1 and 2, it was clear that the zinc oxide particles having a surface coated with organosilicon of an embodiment of the present invention formed an adequately aggregated state in a case where no impact was applied but had dispersibility that allowed dispersion by a light impact. Furthermore, the physical properties of the coating films 1 and 2 were excellent in dispersibility, transmittance, and ultraviolet blocking performance. On the other hand, for Comparative Example 1 in which no hydrolysis was performed, the yield of the surface treatment was poor, and the water repellency was low. For Comparative Example 2 in which the raw material was dried, it was found that the dispersibility was poor due to aggregation caused by drying. For Comparative Examples 3 and 4 in which dry treatment was performed, it was found that the aggregation became more tight and the dispersibility and the transmittance were poor. For Comparative Examples 5 and 6 in which a known product was used, it was found that the dispersibility was poor and the apparent density was low.

### Industrial Applicability

The zinc oxide particles of an embodiment of the present invention can be used as a component for a cosmetic preparation, a dispersion, a heat dissipating filler, a heat dissipating resin composition, a heat dissipating grease, and a heat dissipating coating material composition.

## Claims

1. Zinc oxide particles having a surface coated with organosilicon,
the zinc oxide particles having
a primary particle size of 0.1 µm or less as measured based on a transmission electron micrograph, and
a median size based on a volume of 0.15 µm or less, where the median size is determined by charging the particles in isopropyl alcohol, dispersing the particles by application of ultrasonic wave with a rated output of 600 W and an oscillation amplitude of 100% generated by an ultrasonic homogenizer at a normal temperature for 30 seconds to obtain a dispersion, and measuring the dispersion by a laser diffraction scattering method.

2. The zinc oxide particles having a surface coated with organosilicon according to claim 1, wherein a median size, based on a volume, determined by charging the particles in isopropyl alcohol and stirring at 500 rpm for 5 seconds by a homo disper to obtain a dispersion, and measuring the dispersion by a laser diffraction scattering method is 10 µm or greater.

3. The zinc oxide particles having a surface coated with organosilicon according to claim 1 or 2, wherein an apparent density is 0.5 g/mL or greater.

4. The zinc oxide particles having a surface coated with organosilicon according to any one of claims 1 to 3, wherein a content of silicon is 0.1 mass% or greater and 0.6 mass% or less.

5. The zinc oxide particles having a surface coated with organosilicon according to any one of claims 1 to 4, wherein a median size/primary particle size ratio is 4 or less.

6. A method for producing zinc oxide particles having a surface coated with organosilicon, the method comprising:
a step (1) of preparing a slurry by repulping zinc oxide particles as a raw material in a solvent containing water and an organic solvent;
a step (2) of hydrolyzing an organosilicon compound in a solvent containing water and an organic solvent; and
a step (3) of subjecting the zinc oxide particles to a surface treatment with a silane compound obtained after the hydrolysis.

7. The method for producing zinc oxide particles having a surface coated with organosilicon according to claim 6, wherein the zinc oxide particles as a raw material is produced by a production method comprising a step of aging zinc oxide microparticulates in water containing a zinc salt dissolved therein, and is fed to the step (1) without undergoing a drying step.

8. The method for producing zinc oxide particles having a surface coated with organosilicon according to claim 5 or 6, wherein, for the solvents in the step (1) and the step (2), the organic solvent is contained in an amount of 30 mass% or greater with respect to a total amount of the solvent.

9. The method for producing zinc oxide particles having a surface coated with organosilicon according to any one of claims 6 to 8, wherein the organic solvent contains at least one selected from the group consisting of methanol, ethanol, and propanol.

10. The method for producing zinc oxide particles having a surface coated with organosilicon according to any one of claims 6 to 9, wherein the step (2) is performed in the solvent having a pH adjusted to 3.5 to 4.5.

11. A cosmetic preparation comprising the zinc oxide particles having a surface coated with organosilicon according to any one of claims 1 to 5.

12. A dispersion comprising the zinc oxide particles having a surface coated with organosilicon according to any one of claims 1 to 5.

13. A heat dissipating filler comprising the zinc oxide particles having a surface coated with organosilicon according to any one of claims 1 to 5.

14. A resin composition comprising the zinc oxide particles having a surface coated with organosilicon according to any one of claims 1 to 5.
